(19) Europäisches Patentamt
European Patent Office
Office européen des brevets

(11) Veröffentlichungsnummer : **0 012 200**
**B2**

# (12) NEUE EUROPÄISCHE PATENTSCHRIFT

(45) Veröffentlichungstag der neuen Patentschrift : **05.06.85**

(21) Anmeldenummer : **79104197.3**

(22) Anmeldetag : **29.10.79**

(51) Int. Cl.⁴ : **C 07 C 51/64**, C 07 C 63/10, C 07 C 63/00, C 07 C 63/04, C 07 C 63/22, C 07 C 63/30, C 07 C 65/21

## (54) Verfahren zur Herstellung von farbstabilisierten, aromatischen Carbonsäurechloriden.

(30) Priorität : **08.11.78 DE 2848356**

(43) Veröffentlichungstag der Anmeldung : **25.06.80 Patentblatt 80/13**

(45) Bekanntmachung des Hinweises auf die Patenterteilung : **19.08.81 Patentblatt 81/33**

(45) Bekanntmachung des Hinweises auf die Entscheidung über den Einspruch : **05.06.85 Patentblatt 85/23**

(84) Benannte Vertragsstaaten :
**BE CH DE FR GB IT NL**

(56) Entgegenhaltungen :
**DE-A- 1 109 172**
**DE-A- 2 206 300**
**FR-A- 1 226 245**
**FR-A- 1 502 518**
**FR-A- 2 011 179**
**FR-A- 2 196 305**
**GB-A- 942 621**
**US-A- 3 445 504**
**US-A- 3 557 205**
**Traité de Chimie Organique, Grignard; 1939 tome IX pp. 66 à 69 et 568-569 Masson & Cie Editeurs, Paris**
**Helv. Chim. Acta 42, 1653 (1959) - Dimethylformamid**
**Chemical Abstracts 70, 19607 (1969) - Tetramethylharnstoff**
**Chemical Abstracts 75, 48340m (1971)**

(73) Patentinhaber : **BAYER AG**
**Konzernverwaltung RP Patentabteilung**
**D-5090 Leverkusen 1 Bayerwerk (DE)**

(72) Erfinder : **Böckmann, Walter, Dr.**
**Fabritiusstrasse 19**
**D-4150 Krefeld 11 (DE)**
Erfinder : **Lipper, Karl-August, Dr.**
**Deswatinesstrasse 14**
**D-4150 Krefeld (DE)**
Erfinder : **Brühne, Friedrich, Dr.**
**Fabritiusstrasse 24**
**D-4150 Krefeld 11 (DE)**

**EP 0 012 200 B2**

**Beschreibung**

Die Erfindung betrifft ein Verfahren zur Herstellung von farbstabilisierten, aromatischen Carbonsäurechloriden.

Aromatische Carbonsäurechloride, die durch Umsetzung der entsprechenden aromatischen Carbonsäuren mit Benzotrichlorid erhalten wurden, haben häufig die unangenehme Eigenschaft, sich bei der Lagerung zu verfärben. Die Verfärbung der aromatischen Carbonsäurechloride kann auch nach deren Destillation noch auftreten.

Wird für die weitere Umsetzung der aromatischen Carbonsäurechloride Wert auf deren Farblosigkeit gelegt, so ist eine vorhergehende Destillation erforderlich.

Es wurde nun gefunden, daß man die Verfärbung von aromatischen Carbonsäurechloriden der Formel (I)

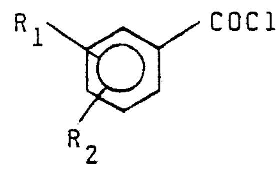

(I)

worin $R_1$ und $R_2$ gleich oder verschieden sein können und für Wasserstoff, Halogen, Alkyl- oder Alkoxyresten mit bis zu 10 C-Atomen stehen,
die durch Umsetzung der entsprechenden aromatischen Carbonsäuren mit Benzotrichlorid erhalten wurden, durch Zusatz eines Stabilisators der Formel (II)

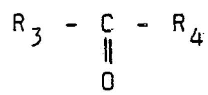

(II)

worin $R_3$ und $R_4$ gleich oder verschieden sein können und für Wasserstoff, gegebenenfalls durch Halogen, niederes Alkyl oder niederes Alkoxy substituierte Alkoxy-, Aryloxy-, Aralkoxy-, Alkyl-, Aralkyl-, Aryl-, Alkenyl-, Mono- oder Dialkylaminoreste stehen, ferner $R_3$ und $R_4$ mit der Carbonylgruppe einen Ring bilden können und wobei $R_3$ und $R_4$ bis zu 12 C-Atome besitzen, oder der Formel (III)

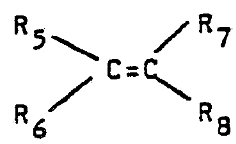

(III)

worin $R_5$ bis $R_8$ gliech oder verschieden sein können und für Wasserstoff, Nitril, Halogen, Carboxyl, gegebenenfalls durch Halogen, niederes Alkyl oder niederes Alkoxy substituierte Carbalkoxy-, Carbaryloxy-, Alkoxy-, Aryloxy-, Acyloxy-, Alkyl-, Aryl-, Aralkyl-, Alkenylreste stehen, ferner $R_5$ und $R_6$ und/oder $R_7$ und $R_8$ oder $R_5$ und $R_7$ und/oder $R_6$ und $R_8$ mit den Atomen, mit denen sie verknüpft sind, einen Ring bilden können und wobei $R_5$ bis $R_8$ bis zu 12 C-Atome besitzen, oder der Formel (IV)

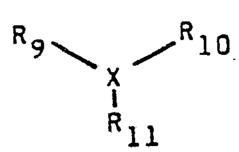

(IV)

worin

$R_9$ bis $R_{11}$ gleich oder verschieden sein können und für Halogen, gegebenenfalls durch Halogen, niederes Alkyl oder niederes Alkoxy substituierte Alkyl-, Aryl-, Alkoxy- oder Aralkoxyreste stehen, und

X Phosphor oder Arsen bedeutet,

in einer Menge von 0,001-2 Gew.-%, bezogen auf die aromatischen Carbonsäurechloride, vermeiden kann.

Ein besonderer Vorteil der erfindungsgemäßen Farbstabilisierung von aromatischen Carbonsäurechloriden ist es, daß zum einen die Stabilisierung dauerhaft ist und zum anderen die farbstabilisierten, aromatischen Carbonsäurechloride überraschenderweise ohne weiteres, d. h. ohne Beeinträchtigung der Reaktivität der aromatischen Carbonsäurechloride, für weitere Umsetzungen, z. B. Friedel-Crafts-Acylierungen, verwendet werden können.

Als Alkyl- oder Alkoxyreste der Formel I kommen beispielsweise solche mit bis zu 10 C-Atomen, bevorzugt bis zu 4 C-Atomen, in Betracht, z. B. Alkylreste, wie Methyl, Äthyl, n-Propyl, iso-Propyl, n-Butyl,

iso-Butyl, tert.-Butyl, n-Pentyl, iso-Pentyl, 2-Methylpentyl, 3-Methylpentyl-, n-Hexyl, n-Heptyl, n-Octyl, iso-Octyl, n-Nonyl, n-Decyl, Cyclohexyl, bevorzugt Methyl, Äthyl, n-Propyl, iso-Propyl, n-Butyl, iso-Butyl, tert.-Butyl ; Alkoxyreste, wie Methoxy, Äthoxy, Propoxy, Butoxy, Pentoxy, Hexoxy, bevorzugt Methoxy und Äthoxy.

Als Halogene seien Fluor, Chlor, Brom, bevorzugt Chlor, genannt.

Bevorzugt werden z. B. folgende aromatische Carbonsäurechloride farbstabilisiert : Benzoylchlorid, o-Toluylsäurechlorid, p-Toluylsäurechlorid, 2-Chlor-benzoylchlorid, 3-Chlor-benzoylchlorid, 4-Chlorbenzoylchlorid, 2,4-Dichlorbenzoylchlorid sowie Anissäurechlorid.

Als Farbstabilisatoren werden solche der allgemeinen Formel (II)

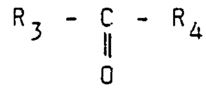

(II)

worin $R_3$ und $R_4$ gleich oder verschieden sein können und für Wasserstoff, gegebenenfalls durch Halogen, niederes Alkyl oder niederes Alkoxy substituierte Alkoxy-, Aryloxy-, Aralkoxy-, Alkyl-, Aralkyl-, Aryl-, Alkenyl-, Mono- oder Dialkylaminoreste stehen, ferner $R_3$ und $R_4$ mit der Carbonylgruppe einen Ring bilden können und wobei $R_3$ und $R_4$ bis zu 12 C-Atome besitzen, für das erfindungsgemäße Verfahren verwendet.

Als Halogene seien Fluor, Chlor, Brom, bevorzugt Chlor, genannt.

Als Reste $R_3$ und $R_4$ in der obengenannten Formel kommen bevorzugt Kohlenwasserstoffreste mit bis zu 8 C-Atomen in Betracht, z. B. Alkoxyreste, wie Methoxy, Äthoxy, Propoxy, Butoxy, tert.-Butoxy, Pentoxy, Hexoxy, bevorzugt Methoxy, Äthoxy ; Aryloxyreste, wie Phenoxy, Chlorphenoxy, Cresyl, bevorzugt Phenoxy ; Aralkoxyreste, wie Benzyloxy, p-Chlorbenzyloxy, p-Tolyloxy, bevorzugt Benzyloxy ; Alkylreste, wie Methyl, Äthyl, n-Propyl, iso-Propyl, n-Butyl, iso-Butyl, tert.-Butyl, n-Pentyl, iso-Pentyl, 2-Methylpentyl, 3-Methylpentyl, n-Hexyl, n-Octyl, iso-Octyl, n-Nonyl, n-Decyl, n-Dodecyl, bevorzugt Methyl, Äthyl, n-Propyl, iso-Propyl ; Aralkylreste, wie Benzyl, p-Chlor-benzyl, p-Methylbenzyl, bevorzugt Benzyl : Arylreste, wie Phenyl, Toluyl, p-Chlorphenyl, p-Methoxyphenyl, bevorzugt Phenyl, Toluyl ; Alkenylreste, wie Vinyl, Propenyl, Allyl, Butenyl-(1), Butenyl-(2), Methallyl, bevorzugt Vinyl, Propenyl, Methallyl ; Mono- und Dialkylaminoreste, wie Methylamino, Äthylamino, Propylamino, Dimethylamino, Diäthylamino, Dipropylamino, bevorzugt Dimethylamino, Diäthylamino ; sowie Alkylenreste, bei denen gegebenenfalls eine oder mehrere Methylengruppen durch Heteroatome ersetzt sein können, wie Äthylen, Trimethylen, Tetramethylen, Pentamethylen, Hexamethylen, 2-Oxatrimethylen, 1-Thiatrimethylen, 1-Iminopentamethylen, bevorzugt Pentamethylen und 1-Iminopentamethylen.

Weiterhin werden als Farbstabilisatoren solche der allgemeinen Formel (III)

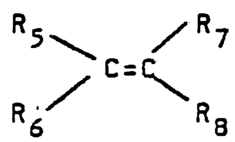

(III)

worin $R_5$ bis $R_8$ gleich oder verschieden sein können und für Wasserstoff, Nitril, Halogen, Carboxyl, gegebenenfalls durch Halogen, niederes Alkyl oder niederes Alkoxy substituierte Carbalkoxy-, Carbaryloxy-, Alkoxy-, Aryloxy-, Acyloxy-, Alkyl-, Aryl-, Aralkyl-, Alkenylreste stehen, ferner $R_5$ und $R_6$ und/oder $R_7$ und $R_8$ oder $R_5$ und $R_7$ und/oder $R_6$ und $R_8$ mit den Atomen, mit denen sie verknüpft sind, einen Ring bilden können und wobei $R_5$ bis $R_8$ bis zu 12 C-Atome besitzen, für das erfindungsgemäße Verfahren verwendet.

Als Halogene seien Fluor, Chlor, Brom, bevorzugt Chlor, genannt.

Als Reste $R_5$ bis $R_8$ in der obengenannten Formel kommen bevorzugt Kohlenwasserstoffreste mit bis zu 8 C-Atomen in Betracht, z. B. Carbalkoxyreste wie Carbomethoxy-, Carbopentoxy-, Carboäthoxy-, Carbopropoxy-, Carbobutoxy-, Carbo-tert. butoxy-, Carbohexoxy-, bevorzugt Carbomethoxy-, Carboäthoxy-, Carbaryloxy-Reste wie Carbophenoxy, Carbochlorphenoxy-, Carbocresyl-, bevorzugt Carbophenoxy-, Alkoxy-Reste wie Methoxy, Äthoxy, Propoxy, Butoxy, tert.-Butoxy, Pentoxy, Hexoxy, bevorzugt Methoxy, Äthoxy ; Aryloxyreste, wie Phenoxy, Chlorphenoxy, Cresyl, bevorzugt Phenoxy ; Acyloxyreste, wie Acetyloxy, Propionyloxy, Butyryloxy, Benzoyloxy, Chlorbenzoyloxy, bevorzugt Acetoxy, Benzoyloxy, Alkylreste, wie Methyl, Äthyl, n-Propyl, iso-Propyl, n-Butyl, iso-Butyl, tert.-Butyl, n-Hexyl, n-Octyl, n-Nonyl, Dodecyl, bevorzugt Methyl, Äthyl, n-Propyl, iso-Propyl ; Aralkylreste, wie Benzyl, p-Chlorbenzyl, p-Methylbenzyl, bevorzugt Benzyl ; Arylreste, wie Phenyl, Toluyl, p-Chlorphenyl, p-Methoxyphenyl, bevorzugt Phenyl, Toluyl ; Alkenylreste, wie Vinyl, Propenyl, Allyl, Butenyl-(1), Butenyl-(2), Methallyl, bevorzugt Vinyl, Allyl, Methallyl ; sowie Alkylenreste, bei denen gegebenenfalls einer oder mehrere Methylengruppen durch Heteroatome ersetzt sein können, wie Äthylen, Trimethylen, Tetramethylen, Pentamethylen, Hexamethylen, 2-Oxatrimethylen, 1-Thiatrimethylen, bevorzugt Tetramethylen und Pentamethylen.

Weiterhin können als Farbstabilisatoren solche der allgemeinen Formel (IV),

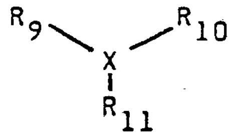

(IV)

worin

$R_9$ bis $R_{11}$ gleich oder verschieden sein können und für Halogen, gegebenenfalls durch Halogen, niederes Alkyl oder niederes Alkoxy substituierte Alkyl-, Aryl-, Alkoxy- oder Aralkoxyreste stehen, und

X Phosphor oder Arsen bedeutet, für das erfindungsgemäße Verfahren verwendet werden.

Als gegebenenfalls substituierte Alkyl-, Aryl-, Alkoxy- oder Aralkoxyreste kommen beispielsweise solche mit bis zu 12 C-Atomen, bevorzugt bis zu 8 C-Atomen, in Betracht, z. B. Alkylreste, wie Methyl, Äthyl, n-Propyl, iso-Propyl, n-Butyl, iso-Butyl, tert.-Butyl, n-Pentyl, n-Hexyl, n-Octyl, n-Nonyl, n-Decyl, n-Dodecyl, bevorzugt Methyl, Äthyl, n-Propyl, iso-Propyl ; Alkoxyreste, wie Methoxy, Äthoxy, Propoxy, Butoxy, Pentoxy, Hexoxy, bevorzugt Methoxy, Äthoxy ; Aralkoxyreste, wie Benzyloxy, p-Chlorbenzyloxy, p-Methylbenzyloxy, bevorzugt Benzyloxy sowie Arylreste wie Phenyl, Toluyl, p-Chlorphenyl, p-Methoxyphenyl, bevorzugt Phenyl und Toluyl.

Als Halogene seien genannt : Fluor, Chlor, Brom, bevorzugt Chlor und Brom.

Bevorzugt werden als Farbstabilisatoren für die aromatischen Carbonsäurechloride verwendet : Aceton, Methyläthylketon, Diäthylketon, Benzaldehyd, Acetophenon, Acetessigester, Acrylsäureäthylester, Methacrylsäuremethylester, Crotonsäure, Vinylacetat, Maleinsäure, Maleinsäurediäthylester, Fumarsäurediäthylester, Dicyclopentadien, ε-Caprolactam, Styrol, Methylvinylketon, Acrolein, Cyclohexen, Allylchlorid, Zimtsäure, Allylalkohol, Acetaldehyd, Triäthylphosphit, Triphenylphosphin, Phosphortrichlorid, Arsentrichlorid, Methacrylsäureamid, Cyclohexanon, wobei Aceton, Cyclohexanon, Dicyclopentadien, Acetessigester, Maleinsäure, ε-Caprolactam, Triäthylphosphit, Phosphortrichlorid besonders bevorzugt sind.

Ganz besonders bevorzugt wird ε-Caprolactam in das erfindungsgemäße Verfahren eingesetzt.

Die genannten Verbindungen der allgemeinen Formel (II), (III), und (IV) können zur Farbstabilisierung der aromatischen Carbonsäurechloride der allgemeinen Formel (I) einzeln oder im Gemisch untereinander verwendet werden, wobei das Mischungsverhältnis keine Rolle für die Wirksamkeit der Stabilisatoren spielt.

Der Zusatz der als Farbstabilisatoren verwendeten Verbindungen der allgemeinen Formel (II), (III), und (IV) zu den aromatischen Carbonsäurechloriden beträgt vorzugsweise 0,01 bis 0,5 Gew.-% und insbesondere 0,02 bis 0,1 Gew.-%.

Es ist auch möglich, die als Farbstabilisatoren verwendeten Verbindungen der allgemeinen Formel (II), (III) und (IV) in einem inerten Lösungmittel gelöst zuzusetzen, wenn dies zweckmäßig erscheint, z. B. zwecks leichter Dosierung oder leichterer Durchmischung, jedoch ist dies im allgemeinen nicht erforderlich. Geeignete inerte Lösungsmittel sind z. B. Benzol, Toluol, Xylol, Cyclohexan und Tetrachlorkohlenstoff.

Die Konzentration der Lösung der Farbstabilisatoren im Lösungsmittel wird zweckmäßig so konzentriert gewählt, daß sie an der oberen Grenze der Löslichkeit der Farbstabilisatoren liegt, um die sich ergebende Konzentration des Lösungsmittels in den aromatischen Carbonsäurechloriden so gering wie möglich zu halten, z. B. wird für das System Toluol-Phosphortrichlorid ein Gew.-Verhältnis von 50 zu 50 bevorzugt.

## Beispiel 1

In Benzoylchlorid (100 g) wird Aceton (0,05 g) gelöst. Die Probe wird im Dunkeln gelagert. Die Mischung ist noch nach Monaten farblos. Dagegen hatte eine umbehandelte Probe des gleichen Materials eine Farbzahl von 150 APHA. APHA bedeutet Hazen-Farbzahl gemäß DIN 53 409.

## Beispiel 2

In Benzoylchlorid (100 g) wird ε-Caprolactam (0,039) gelöst. Die Probe wird im Dunkeln gelagert.

Nach mehrmonatiger Lagerung ist die Probe noch farblos. Eine unbehandelte Probe des gleichen Materials hatte eine Farbzahl von 150 APHA.

## Beispiel 3

In Benzoylchlorid (100 g) wird Dicyclopentadien (0,01 g) gelöst. Nach mehrmonatiger Lagerung im Dunkeln ist die Probe noch farblos. Eine Vergleichsprobe des gleichen Materials hatte eine Farbzahl von 150 APHA.

## Beispiel 4

In o-Toluylsäurechlorid (100 g) wird Acetophenon (0,02 g) gelöst. Die Probe ist auch nach

mehrmonatiger Lagerung im Dunkeln noch farblos. Ein Vergleichsmuster der gleichen Materials hatte eine Farbzahl von 100 APHA.

Beispiel 5

In Benzoylchlorid (100 g) wird Triäthylphosphit (0,05 g) gelöst. Die Probe ist nach mehrmonatiger Lagerung im Dunkeln noch farblos. Eine Vergleichsprobe des gleichen Materials hatte eine Farbzahl von 150 APHA.

Beispiel 6

In Benzoylchlorid (100 g) mit einer Farbzahl von 200 APHA wird ε-Caprolactam (0,05 g) gelöst. Nach 4 Stunden ist die Probe farblos (APHA < 15).

Beispiel 7

In Benzoylchlorid (100 g) wird Malonsäurediäthylester (0,02 g) gelöst. Nach mehrmonatiger Lagerung im Dunkeln ist das Produkt noch farblos. Eine Vergleichsprobe des gleichen Materials hatte eine Farbzahl von 150 APHA.

Beispiel 8

In Benzoylchlorid (100 g) mit einer Farbzahl von 100 APHA wird Cyclohexen (0,05 g) gelöst. Nach 2 Stunden ist die Probe farblos (10 APHA).

Entsprechend der in den Beispielen 1-8 dargelegten Arbeitsweise wurden weitere Beispiele ausgeführt, die in den Tabellen 1-3 zusammengestellt sind.

Tabelle 1

| Beispiel Nr. | Säurechlorid | Stabilisator- -formel | -name | menge (%) | Farbzahl (APHA) mit Stabilisator | ohne Stabilisator |
|---|---|---|---|---|---|---|
| 9 | Benzoylchlorid | II | Aceton | 0,05 | 5 | 150 |
| | | II | Acetophenon | 0,02 | 20 | 90 |
| | | II | Acetaldehyd | 0,01 | 10 | 80 |
| | | II | Benzaldehyd | 0,5 | 20 | 90 |
| | | II | Acrolein | 0,2 | 10 | 90 |
| | | II | ε-Caprolactam | 0,03 | 10 | 150 |
| | | II | Laurinlactam | 0,1 | 20 | 90 |
| | | II | Cyclohexanon | 0,1 | 10 | 150 |
| 10 | p-Chlorbenzoylchlorid | II | Aceton | 0,2 | 20 | > 100 |
| | | II | ε-Caprolactam | 0,2 | 20 | > 100 |
| 11 | O-Toluylsäurechlorid | II | Aceton | 0,2 | 20 | > 100 |
| | | II | ε-Caprolactam | 0,2 | 20 | > 100 |
| 12 | Anissäurechlorid | II | Aceton | 0,2 | 20 | > 100 |
| | | II | ε-Caprolactam | 0,2 | 20 | > 100 |

(Siehe Tabelle 2 Seite 6 f.)

Tabelle 2

| Beispiel Nr. | Säurechlorid | Stabilisator- -formel | -name | menge (%) | Farbzahl (APHA) mit Stabilisator | ohne Stabilisator |
|---|---|---|---|---|---|---|
| 13 | Benzoylchlorid | III | Cyclohexen | 0,05 | 10 | 100 |
| | | III | Allylalkohol | 0,5 | 20 | 90 |
| | | III | Dicyclopentadien | 0,01 | 10 | 150 |
| | | III | Undecensäure | 0,5 | 40 | 100 |
| | | II | Maleinsäure | 0,5 | 10 | 90 |
| | | II | Fumarsäure | 0,5 | 10 | 90 |
| | | II | Acrylsäuremethylester | 0,5 | 10 | 70 |
| | | III | Vinylacetat | 0,5 | 20 | 90 |
| | | II | Zimtsäure | 0,5 | 30 | 90 |
| 14 | m-Chlor-benzoylchlorid | III | Cyclohexen | 0,5 | 10 | 100 |
| 15 | O-Toluylsäurechlorid | III | Cyclohexen | 0,5 | 20 | > 100 |
| 16 | Anissäurechlorid | III | Cyclohexen | 0,5 | 20 | > 100 |

Tabelle 3

| Beispiel Nr. | Säurechlorid | Stabilisator- -formel | -name | menge (%) | Farbzahl (APHA) mit Stabilisator | ohne Stabilisator |
|---|---|---|---|---|---|---|
| 17 | Benzoylchlorid | IV | Triäthylphosphit | 0,3 | 10 | 100 |
| 18 | O-Toluylsäurechlorid | IV | Triäthylphosphit | 0,3 | 20 | > 100 |
| 19 | p-Chlor-benzoylchlorid | IV | Triäthylphosphit | 0,3 | 20 | 100 |
| 20 | Anissäurechlorid | IV | Triäthylphosphit | 0,3 | 20 | > 100 |
| 21 | Benzoylchlorid | IV | Phosphortrichlorid | 0,2 | 10 | 150 |
| 22 | o-Toluylsäurechlorid | IV | Phosphortrichlorid | 0,2 | 20 | > 100 |
| 23 | p-Chlor-benzoylchlorid | IV | Phosphortrichlorid | 0,2 | 10 | 100 |
| 24 | Anissäurechlorid | IV | Phosphortrichlorid | 0,2 | 20 | > 100 |
| 25 | Benzoylchlorid | IV | Arsentrichlorid | 0,3 | 10 | 150 |

**Patentansprüche**

1. Verfahren zur Stabilisierung von aromatischen Carbonsäurechloriden der Formel

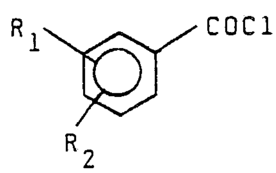

worin $R_1$ und $R_2$ gleich oder verschieden sein können und für Wasserstoff, Halogen, Alkyl- oder Alkoxyresten mit bis zu 10 C-Atomen stehen,
die durch Umsetzung der entsprechenden aromatischen Carbonsäuren mit Benzotrichlorid erhalten wurden, gegen Verfärbung, dadurch gekennzeichnet, daß man 0,001 bis 2 Gew.-% eines Stabilisators der Formel

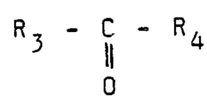

worin $R_3$ und $R_4$ gleich oder verschieden sein können und für Wasserstoff, gegebenenfalls durch Halogen, niederes Alkyl oder niederes Alkoxy substituierte Alkoxy-, Aryloxy-, Aralkoxy-, Alkyl-, Aralkyl-, Aryl-, Alkenyl-, Mono- oder Dialkylaminoreste stehen, ferner $R_3$ und $R_4$ mit der Carbonylgruppe einen Ring bilden können und wobei $R_3$ und $R_4$ bis zu 12 C-Atome besitzen, oder der Formel

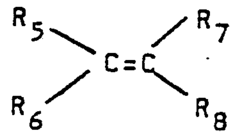

worin $R_5$ bis $R_8$ gleich oder verschieden sein können und für Wasserstoff, Nitril, Halogen, Carboxyl, gegebenenfalls durch Halogen, niederes Alkyl oder niederes Alkoxy substituierte Carbalkoxy-, Carbaryloxy-, Alkoxy-, Aryloxy-, Acyloxy-, Alkyl-, Aryl-, Aralkyl-, Alkenylreste stehen, ferner $R_5$ und $R_6$ und/oder $R_7$ und $R_8$ oder $R_5$ und $R_7$ und/oder $R_6$ und $R_8$ mit den Atomen, mit denen sie verknüpft sind, einen Ring bilden können und wobei $R_5$ bis $R_8$ bis zu 12 C-Atome besitzen, oder der Formel

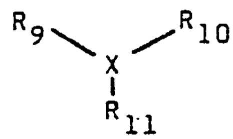

worin

$R_9$ bis $R_{11}$ gleich oder verschieden sein können und für Halogen, gegebenenfalls durch Halogen, niederes Alkyl oder niederes Alkoxy substituierte Alkyl-, Aryl-, Alkoxy- oder Aralkoxyreste stehen, und

X Phosphor oder Arsen bedeutet,

bezogen auf die aromatischen Carbonsäurechloride, zusetzt.

2. Verfahren nach Anspruch 1, dadurch gekennzeichnet, daß man 0,01 bis 0,5 Gew.-% eines Stabilisators zusetzt.

3. Verfahren nach Ansprüchen 1 bis 2, dadurch gekennzeichnet, daß man die als Farbstabilisatoren genannten Verbindungen im Gemisch untereinander zusetzt.

4. Verfahren nach Ansprüchen 1 bis 3, dadurch gekennzeichnet, daß man die als Farbstabilisatoren genannten Verbindungen gelöst in einem inerten Lösungsmittel zusetzt.

5. Verfahren nach Ansprüchen 1 bis 4, dadurch gekennzeichnet, daß man als Stabilisatoren Aceton, Acetessigester, Dicyclopentadien, Maleinsäure, Cyclohexanon, ε-Caprolactam, Phosphortrichlorid und/oder Triethylphosphit zusetzt.

## Claims

1. Process for stabilising aromatic carboxylic acid chlorides of the formula

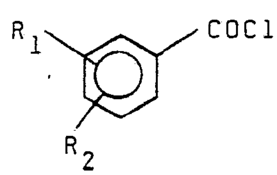

wherein $R_1$ und $R_2$ can be identical or different and represent hydrogen, halogen, or alkyl or alkoxy radicals with up to 10 C atoms,

which have been obtained by the reaction of the corresponding aromatic carboxylic acids with benzotrichloride, against discolouration, characterised in that 0.001 to 2 % by weight of a stabiliser of the formula

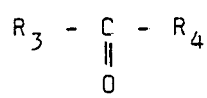

wherein $R_3$ und $R_4$ can be identical or different and represent hydrogen, or alkoxy, aryloxy, aralkoxy, alkyl, aralkyl, aryl, alkenyl or mono- or dialkylamino radicals which are optionally substituted by halogen, lower alkyl or lower alkoxy, or, furthermore, $R_3$ and $R_4$, with the carbonyl group, can form a ring, $R_3$ and $R_4$ having up to 12 C atoms, or of the formula

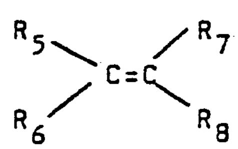

wherein $R_5$ to $R_8$ can be identical or different and represent hydrogen, nitrile, halogen, carboxyl, or carbalkoxy, carbaryloxy, alkoxy, aryloxy, acyloxy, alkyl, aryl, aralkyl or alkenyl radicals which are optionally substituted by halogen, lower alkyl or lower alkoxy, or, furthermore, $R_5$ and $R_6$ and/or $R_7$ and $R_8$ or $R_5$ and $R_7$ and/or $R_6$ and $R_8$, with the atoms to which they are linked, can form a ring, $R_5$ to $R_8$ having up to 12 C atoms, or of the formula

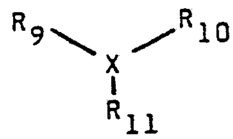

wherein

$R_9$ to $R_{11}$ can be identical or different and represent halogen, or alkyl, aryl, alkoxy or aralkoxy radicals which are optionally substituted by halogen, lower alkyl or lower alkoxy, and

X denotes phosphorus or arsenic, based on the aromatic carboxylic acid chlorides, is added.

2. Process according to Claim 1, characterised in that 0.01 to 0.5 % by weight of a stabiliser is added.

3. Process according to Claims 1 to 2, characterised in that the compounds mentioned as colour stabilisers are added in admixture with one another.

4. Process according to Claims 1 to 3, characterised in that the compounds mentioned as colour stabilisers are added dissolved in an inert solvent.

5. Process according to Claims 1 to 4, characterised in that acetone, ethyl acetoacetate, dicyclopentadiene, maleic acid, cyclohexanone, ε-caprolactam, phosphorus trichloride and/or triethyl phosphite are added as stabilisers.

**Revendications**

1. Procédé pour la stabilisation contre la coloration de chlorures d'acides carboxyliques aromatiques de formule

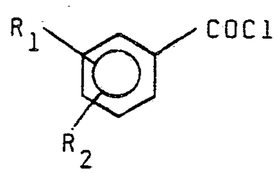

dans laquelle $R_1$ et $R_2$ peuvent être identiques ou différents et représentent l'hydrogène, un halogène, des restes alkyles ou alcoxy jusqu'en $C_{10}$,

qui ont été obtenus par réaction des acides carboxyliques aromatiques avec le chlorure de benzényle, caractérisé en ce que l'on ajoute 0,001 à 2 % en poids d'un stabilisant de formule

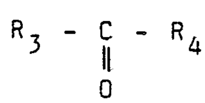

dans laquelle $R_3$ et $R_4$ peuvent être identiques ou différents et représentent l'hydrogène, des restes alcoxy, aryloxy, arylalcoxy, alkyles, arylalkyles, aryles, alcényles, mono- ou dialkylamino, éventuellement substitués par un halogène, un groupe alkyle inférieur ou un groupe alcoxy inférieur ; $R_3$ et $R_4$ peuvent en outre former un noyau avec le groupe carbonyle et $R_3$ et $R_4$ ont jusqu'à 12 atomes de carbone ; ou de formule

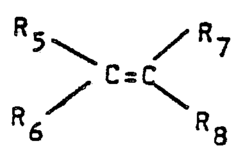

dans laquelle $R_5$ à $R_8$ peuvent être identiques ou différents et représentent l'hydrogène, un groupe nitrile, un halogène, un groupe carboxyle, des restes carbalcoxy, carbaryloxy, alcoxy, aryloxy, acyloxy, alkyles, aryles, arylalkyles, alcényles éventuellement susbstitués par un halogène ; un groupe alkyle inférieur, ou un groupe alcoxy inférieur, $R_5$ et $R_6$ et/ou $R_7$ et $R_8$ ou $R_5$ et $R_7$ et/ou $R_6$ et $R_8$ peuvent en outre former un noyau avec les atomes auxquels ils sont liés et $R_5$ à $R_8$ ont jusqu'à 12 atomes de carbone, ou de formule

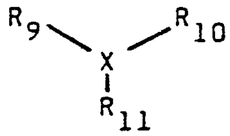

dans laquelle

$R_9$ à $R_{11}$ peuvent être identiques ou différents et représentent un halogène, des restes alkyles, aryles, alcoxy ou arylalcoxy éventuellement substitués par un halogène, un groupe alkyle inférieur ou un groupe alcoxy inférieur ; et

X représente le phosphore ou l'arsenic, par rapport aux chlorures d'acides carboxyliques aromatiques.

2. Procédé selon la revendication 1, caractérisé en ce qu'on ajoute 0,01 à 0,5 % en poids d'un stabilisant.

3. Procédé selon les revendications 1 et 2, caractérisé en ce que l'on ajoute les composés mentionnés comme stabilisants de couleur en mélange entre eux.

4. Procédé selon les revendications 1 à 3, caractérisé en ce que l'on ajoute les composés mentionnés comme stabilisants de couleur dissous dans un solvant inerte.

5. Procédé selon les revendications 1 à 4, caractérisé en ce que l'on ajoute comme stabilisants l'acétone, l'acétylacétate d'éthyle, le dicyclopentadiène, l'acide maléique la cyclohexanone, l'ε-caprolactame, le trichlorure de phosphore et/ou le phosphite de triéthyle.